# EUROPEAN PATENT APPLICATION

(11) **EP 4 843 669 A1**
(43) Date of publication of application: **25.11.2026**
(21) Application number: 25186918.6
(22) Date of filing: 02.07.2025
(51) Int. Cl.: B01L 3/02

(54) **LIQUID SAMPLING DEVICE**

(30) Priority: 20.05.2025 CN 202510646563; 20.05.2025 CN 202520999479 U
(71) Applicant: URIT Medical Electronic Co., Ltd., 541004 Guilin Guangxi (CN)
(72) Inventor: LI, Qiuping, Guilin, Guangxi, 541004 (CN); PAN, Nengkun, Guilin, Guangxi, 541004 (CN); WU, Haocheng, Guilin, Guangxi, 541004 (CN); HUANG, Qiong, Guilin, Guangxi, 541004 (CN); LIU, Jiasong, Guilin, Guangxi, 541004 (CN)
(74) Representative: Ottazzo, Marco Francesco Agostino

(57) **Abstract**

The present disclosure relates to the technical field of medical detection, and in particular to a liquid sampling device. The liquid sampling device comprises a gripping portion and a sampling portion, wherein the sampling portion is connected to the gripping portion, the sampling portion is provided with a capillary channel, a slit is provided on a side wall of the sampling portion, a through hole is provided at one end of the sampling portion away from the gripping portion, and the through hole and the slit are both in communication with the capillary channel. The liquid sampling device can achieve multiple suctions to obtain sufficient liquid samples and improve the accuracy of detection.

## Description

### Technical field

The present disclosure relates to the technical field of medical detection, and in particular to a liquid sampling device.

### Background art

In professional fields such as biomedical research, environmental monitoring and chemical analysis, liquid sampling is a key link in experimental detection, and its accuracy and safety directly affect the reliability of experimental results.

However, existing liquid sampling devices still have several technical limitations in practical applications. First, bubbles are easily mixed into the sampling channel, resulting in insufficient actual sampling volume, which in turn affects the accuracy of the test results. In addition, it is impossible to achieve multiple suction and automatic exhaust functions at the same time.

### Summary of the invention

The objective of the present disclosure includes providing a liquid sampling device which can discharge bubbles and can achieve multiple suction to obtain sufficient liquid samples and improve the accuracy of detection.

The embodiment of the present disclosure can be implemented as follows.

The present disclosure provides a liquid sampling device, comprising:
- a gripping portion; and
- a sampling portion,
wherein the sampling portion is connected to the gripping portion, the sampling portion is provided with a capillary channel, a slit is provided on a side wall of the sampling portion, and a through hole is provided at one end of the sampling portion away from the gripping portion, and wherein the through hole and the slit are both in communication with the capillary channel.

In an optional embodiment, an extension direction of the slit is parallel to an axis of the capillary channel.

In an optional embodiment, the slit runs through both ends of the sampling portion.

In an optional embodiment, two inner walls of the slit, which are opposite to each other, are provided at an angle, so that a width of the slit gradually increases from the capillary channel toward the outside.

In an optional embodiment, the angle is α, where 15° ≤ α ≤ 120°.

In an optional embodiment, the liquid sampling device also includes an abutment portion, and the abutting portion is connected to one end of the sampling portion close to the gripping portion.

In an optional embodiment, the abutting portion is provided with an exhaust hole, the exhaust hole is in communication with the capillary channel, and a diameter of the exhaust hole is smaller than a diameter of the through hole.

In an optional embodiment, the gripping portion is provided with an exhaust groove, and the exhaust groove is in communication with the exhaust hole.

In an optional embodiment, a width of the slit is b, where 0.01 mm ≤ b ≤ 0.5 mm.

In an optional embodiment, the capillary channel includes a collection section and a flow limiting section that are in communication to each other, the collection section is in communication with the through hole, and a diameter of the flow limiting section is smaller than a diameter of the collection section.

The beneficial effects of the liquid sampling device provided by the embodiment of the present disclosure include the following.

The liquid sampling device provided in this embodiment of the present disclosure is provided a gripping portion for gripping and taking, and liquid is collected through the capillary channel of the sampling portion, so that the liquid can enter the capillary channel through the through hole, so as to transfer the liquid sample in the capillary channel to the detection device for detection. The liquid sampling device provides a slit, so that the bubbles in the capillary channel can be discharged through the slit during the sampling process, and therefore enough liquid samples can be collected, thus improving the accuracy of the detection. The present embodiment can achieve multiple sampling and automatic bubble discharge by providing the capillary channel and the slit.

### Brief description of drawings

In order to more clearly illustrate the technical solution of the embodiment of the present disclosure, the following will briefly introduce the drawings required for use in the embodiment. It should be understood that the following drawings only show some embodiments of the present disclosure, and therefore should not be regarded as limiting the scope. For those ordinary skilled in the art, other relevant drawings can also be obtained based on these drawings without inventive work.
Figure 1 is a structural schematic diagram of a liquid sampling device provided in the embodiment at a first viewing angle;
Figure 2 is a structural schematic diagram of the liquid sampling device provided in the embodiment at a second viewing angle;
Figure 3 is a sectional schematic diagram at A-A in Figure 2;
Figure 4 is a sectional schematic diagram of the liquid sampling device provided in the embodiment; and
Figure 5 is a sectional schematic diagram of the liquid sampling device provided in other embodiments.

### Detailed description of embodiments

In order to make the objective, technical solution and advantages of the embodiment of the present disclosure clear, the technical solution in the embodiment of the present disclosure will be clearly and completely described below in combination with drawings in the embodiment of the present disclosure. Obviously, the described embodiments are a part of the embodiments of the present disclosure, not all of the embodiments. The components of the embodiment of the present disclosure generally described and shown in the drawings here can be arranged and designed in various different configurations.

Therefore, the following detailed description of the embodiment of the present disclosure provided in the drawings is not intended to limit the scope claimed the application, but only represents the selected embodiment of the present disclosure. Based on the embodiments of the present disclosure, all other embodiments obtained by those ordinary skilled in the art without inventive work are within the scope of protection of the present disclosure.

It should be noted that similar numbers and letters represent similar items in the following drawings, so when an item is defined in one drawing, it does not need to be further defined and explained in the subsequent drawings.

In the description of the present disclosure, it should be noted that if the terms "upper", "lower", "inner", "outer" and the like indicate an orientation or positional relationship based on the orientation or positional relationship shown in the drawings, or the orientation or positional relationship in which the product of the present disclosure is usually placed when in use. It is only for the convenience of describing the present disclosure and simplifying the description, and does not indicate or imply that the device or element referred to must have a specific orientation, be constructed and operated in a specific orientation, and therefore cannot be understood as a limitation of the present disclosure.

In addition, if the terms "first", "second" and the like appear, they are only used to distinguish the description, and cannot be understood as indicating or implying relative importance.

It should be noted that the features in the embodiments of the present disclosure can be combined with each other without conflict.

In the prior art, during the process of multiple intermittent sample aspiration by the liquid sampling device, bubbles are easily mixed into the sampling pipeline, resulting in insufficient actual sampling volume, thereby affecting the accuracy of the test results; secondly, the existing sampler has a single function and cannot simultaneously realize multiple aspiration and automatic exhaust functions.

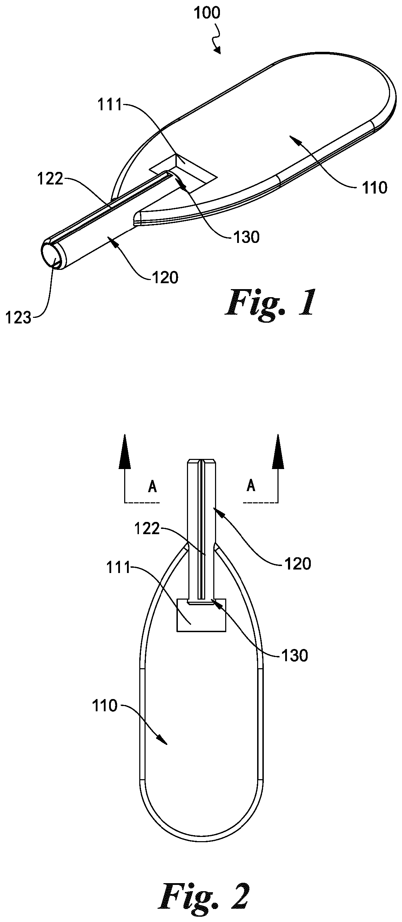

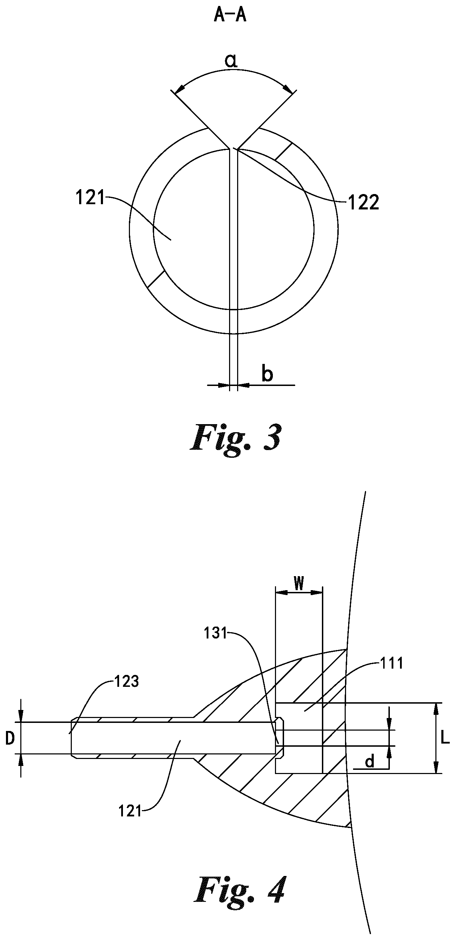

In view of the above problems, referring to Figure 1 to Figure 4, the present disclosure provides a liquid sampling device 100. The liquid sampling device 100 includes a gripping portion 110 and a sampling portion 120, which are connected to each other. The staff can hold the gripping portion 110 with his/her hand and then collect liquid through the sampling portion 120. It can be understood that the gripping portion 110 can also be clamped by a mechanical arm to drive the liquid sampling device 100 to move, so as to bring the sampling portion 120 close to the liquid to achieve automatic sampling.

Since the staff needs to hold the gripping portion 110 with his/her hand, the edge of the gripping portion 110 should be designed to be an arc shape to improve the comfort of the staff holding it.

The sampling portion 120 in this embodiment is provided with a capillary channel 121, and a through hole 123 is provided at one end of the sampling portion 120 away from the gripping portion 110, so that the end where the through hole 123 is located can contact the liquid, and the liquid will not contact the gripping portion 110.

It can be understood that the capillary channel 121 is made of hydrophilic material and has a smooth inner wall surface. In addition, the diameter of the capillary channel 121 is small, so that when the end of the sampling portion 120 contacts the liquid, the sampling portion 120 can use the tension and viscosity of the liquid to allow the liquid to flow from the through hole 123 into the capillary channel 121, thereby collecting the liquid.

In addition, in this embodiment, the sampling portion 120 is also provided with a slit 122, and the capillary channel 121 is in communication with the outside world through the slit 122. In the process of collecting liquid, bubbles will be mixed in. Since the slit 122 is provided in this embodiment, the bubbles can be discharged from the slit 122 after entering the capillary channel 121, thereby enabling the liquid sampling device 100 to collect enough liquid samples to improve the accuracy of the detection.

It should be noted that the slit 122 in this embodiment has a very small width, and the liquid has tension and viscosity. Therefore, the liquid will not leak from the slit 122, and only bubbles will be discharged from the slit 122. Similarly, after the end of the sampling portion 120 leaves the liquid, the liquid sample in the capillary channel 121 will not flow out from the through hole 123, and when the end of the sampling portion 120 contacts the liquid again, the liquid can continue to enter the capillary channel 121 from the through hole 123. Therefore, even if the liquid has not yet filled the capillary channel 121 , if the sampling portion 120 leaves the surface of the liquid to be collected, it can continue to collect by contacting again. Thus, this embodiment can achieve multiple suction of liquid and automatic exhaust.

According to the above description, please refer to Figures 1 to Figure 4. The width of the slit 122 in this embodiment is b, wherein the value range of the width of the slit 122 is 0.01 mm ≤ b ≤ 0.5 mm. The width of the slit 122 can be adjusted according to the liquid to be collected. Since the tension and viscosity of different liquids are different, the width of the slit 122 should also be different for different liquids.

Specifically, if the liquid to be collected is blood, the viscosity of blood is different from that of ordinary liquid due to the different compression volume of blood. At this time, the width of the slit 122 should be 0.05mm-0.1mm, so as to ensure that blood will not overflow from the slit 122, and gas can be discharged from the slit 122, thereby avoiding bubbles being mixed into the collected blood. If the liquid to be collected is urine, the viscosity of urine is low, so the width of the slit 122 should be about 0.05mm to avoid urine overflowing from the slit 122.

The operation process of the liquid sampling device 100 provided in this embodiment is as follows.

The staff holds the gripping portion 110 with his/her hand, drives the liquid sampling device 100 close to the liquid to be collected, and makes the end of the sampling portion 120 where the through hole 123 is located to contact with the liquid. After contacting the end, the liquid enters the capillary channel 121 through the through hole 123.

After the liquid fills the whole capillary channel 121, the liquid sampling device 100 is driven close to the detection device, and the end of the sampling portion 120 where the through hole 123 is located is contacted with the detection device. Since the balance of inner and outer tension of the liquid is broken at this time, the liquid in the capillary channel 121 will be discharged from the through hole 123 to enter the detection device. Then, the detection device can be used to detect the collected liquid sample.

It should be noted that since the volume of the capillary channel 121 will not change, and the liquid needs to fill the capillary channel 121 before the collection is completed, the volume of the collected liquid is equal to the volume of the capillary channel 121, so this embodiment can achieve quantitative collection.

According to the above description, after the liquid sampling device 100 provided in this embodiment contacts the liquid, the liquid will automatically enter the capillary channel 121. Compared with the way of collecting liquid using a dropper in the prior art, the volume of the collected liquid is more controllable and can also avoid liquid contamination.

In this embodiment, the extension direction of the slit 122 in this embodiment is parallel to the axis of the capillary channel 121. It should be noted that both ends of the capillary channel 121 in this embodiment are open ends. In order to ensure that the bubbles can be discharged no matter where they move to, the slit 122 in this embodiment runs through both ends of the sampling portion 120, that is, the slit 122 extends from one end of the sampling portion 120 to the other end of the sampling portion 120, so that the length of the slit 122 is equal to the length of the capillary channel 121. Therefore, in the process of liquid entering the capillary channel 121, no matter where the bubbles appear in the liquid, they can be discharged from the slit 122 to ensure that the collected liquid sample does not contain bubbles. It is ensured that the volume of the collected liquid is sufficient, and the accuracy of the detection is improved.

In other embodiments, the slit 122 can also be provided in multiple sections, and the multiple sections can be arranged in sequence along the axial direction of the capillary channel 121, and the extension direction of each section can overlap or not overlap and be staggered. In other embodiments, the slit 122 can also be provided around the sampling portion 120 in a winding manner.

Further, please refer to Figure 1 to Figure 4. Since the volume of the liquid sampling device 100 is small and the width of the slit 122 is small, in order to facilitate the opening of the slit 122 on the sampling portion 120, the two inner walls opposite to each other of the slit 122 in this embodiment are provided at an angle, so that the width of the slit 122 gradually increases from the capillary channel 121 toward the outside.

In this embodiment, the angle is α, wherein the value range of the angle is 15° ≤ α ≤ 120°, and the value of the angle can be adjusted according to the width of the slit 122 to be opened. Specifically, the angle can be 20°, 70° and 100°.

It should be noted that when the two inner walls opposite to each other of the slit are provided at an angle, the width of the inner wall is smaller than the width of the outer wall. At this time, since the width of the inner wall will affect the flow of the liquid in the capillary channel 121, the width of the slit 122 refers to the width of the inner wall.

In other embodiments, two inner walls opposite to each other of the slit 122 can also be parallel to each other, so that the width of the outer wall is equal to the width of the inner wall.

Further, referring from Figure 1 to Figure 4, this embodiment also includes an abutment portion 130 connected to one end of the sampling portion 120 close to the gripping portion 110, and the abutment portion 130 is provided with an exhaust hole 131, and the exhaust hole 131 is in communication with the capillary channel 121, that is, the abutment portion 130 in this embodiment is a ring structure. In this embodiment, the diameter of the through hole 123 is D, and the diameter of the exhaust hole 131 is d, where d < D.

It can be understood that since the diameter of the exhaust hole 131 is smaller than the diameter of the through hole 123, the pressure between the two ends of the capillary channel 121 is different, resulting in a pressure difference and further resulting in a change in the resistance received by the liquid when it flows. Therefore, this embodiment can control the flow rate of the liquid through the exhaust hole 131.

According to the above description, the gripping portion 110 is provided with an exhaust groove 111 in communication with the exhaust hole 131. It is understandable that after the liquid is absorbed in the capillary tube 121, if the liquid sampling device 100 is inverted, due to the provision of the exhaust hole 131, and the liquid has tension, the tension of the liquid can be used to resist gravity, thereby preventing the liquid from overflowing from the exhaust hole 131.

However, the diameter of the exhaust hole 131 is smaller than the diameter of the through hole 123, resulting in different capillary pressures at both ends, which affects the tension on the surface of the liquid. Therefore, the exhaust groove 111 is provided in this embodiment to balance the capillary pressures at both ends, thereby preventing the liquid from overflowing from the exhaust hole 131 and flowing to the gripping portion 110, thereby preventing the staff from contacting the liquid.

It is understandable that the exhaust hole 131 constitutes the first layer of protection, and the exhaust groove 111 constitutes the second layer of protection, which together prevent the liquid from flowing to the gripping portion 110.

It should be noted that after the liquid overflows the exhaust hole 131 due to its own gravity, the exhaust groove 111 is provided in this embodiment, so that the capillary pressures at both ends of the sampling portion 120 are balanced. In addition, the liquid has viscosity, so that the liquid can adhere to the periphery of the exhaust hole 131, that is, the end surface of the sampling portion 120. Therefore, it will not flow to the gripping portion 110.

In addition, in order to ensure that the exhaust groove 111 can balance the capillary pressure, the length and width of the exhaust groove 111 need to be adjusted according to the diameter of the capillary channel 121. In this embodiment, the diameter of the capillary channel 121 is D, the length of the exhaust groove 111 is L, and the width of the exhaust groove 111 is W, wherein the length of the exhaust groove 111 has a value range of: 0.5D ≤ L ≤ 1.5D, and the width of the exhaust groove 111 has a value range of: 0.5D ≤ W ≤ 1.5D.

In other embodiments, the abutment portion 130 can also be configured to close the end of the capillary channel 121, so that only one end of the capillary channel 121 is provided with a hole for liquid to enter and discharge.

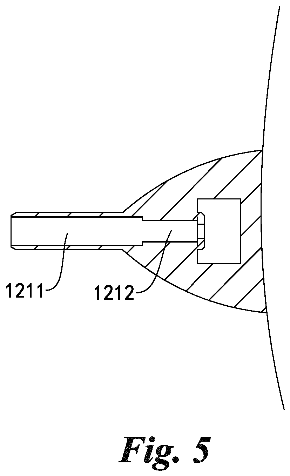

Further, it should be noted that the capillary channel 121 in this embodiment is cylindrical, and its diameter does not change. Referring to Figure 5, in other embodiments, the capillary channel 121 can include a collection section 1211 and a flow limiting section 1212 that are in communication to each other. The collection section 1211 is in communication with the through hole 123, and the flow limiting section 1212 is in communication with the exhaust hole 131. That is, the collection section 1211 is the front end, and the flow limiting section 1212 is the tail end. The diameter of the flow limiting section 1212 is smaller than the diameter of the collection section 1211, so that when the liquid flows from the collection section 1211 into the flow limiting section 1212, the flow rate decreases, thereby adjusting the flow rate of the liquid.

In summary, the present embodiment provides a gripping portion 110 for gripping and taking, and liquid is collected through the capillary channel 121 of the sampling portion 120, so that the liquid can enter the capillary channel 121 through the through hole 123, so as to transfer the liquid sample in the capillary channel 121 to the detection device for detection. The present embodiment provides a slit 122, so that the bubbles in the capillary channel 121 can be discharged through the slit 122 during the sampling process, and therefore enough liquid samples can be collected to improve the accuracy of the detection. The present embodiment can achieve multiple sampling and automatic bubble discharge by providing the capillary channel 121 and the slit 122.

The above is only a specific embodiment of the present disclosure, but the protection scope of the present disclosure is not limited thereto. Any changes or replacements that can be easily thought of by those skilled familiar with the technical field within the technical scope disclosed by the present disclosure should be covered within the protection scope of the present disclosure.

### Reference signs

100: liquid sampling device;
110: gripping portion;
111: exhaust groove;
120: sampling portion;
121; capillary channel;
1211: collection section;
1212: flow limiting section;
122: slit;
123: through hole;
130: abutment portion;
131: exhaust hole.

## Claims

1. A liquid sampling device (100), comprising:
- a gripping portion (110); and
- a sampling portion (120),
wherein the sampling portion (120) is connected to the gripping portion (110), the sampling portion (120) is provided with a capillary channel (121), a slit (122) is provided on a side wall of the sampling portion (120), and a through hole (123) is provided at one end of the sampling portion (120) away from the gripping portion (110), and wherein the through hole (123) and the slit (122) are both in communication with the capillary channel (121).

2. The liquid sampling device (100) according to claim 1, wherein an extension direction of the slit (122) is parallel to an axis of the capillary channel (121).

3. The liquid sampling device (100) according to claim 1, wherein the slit (122) runs through both ends of the sampling portion (120).

4. The liquid sampling device (100) according to claim 1, wherein two inner walls of the slit (122), which are opposite to each other, are arranged at an angle, so that a width of the slit (122) gradually increases from the capillary channel (121) toward the outside.

5. The liquid sampling device (100) according to claim 4, wherein the angle is α, where 15° ≤ α ≤ 120°.

6. The liquid sampling device (100) according to claim 1, wherein the liquid sampling device (100) further comprises an abutment portion (130), and the abutting portion (130) is connected to one end of the sampling portion (120) close to the gripping portion (110).

7. The liquid sampling device (100) according to claim 6, wherein the abutment portion (130) is provided with an exhaust hole (131), the exhaust hole (131) is in communication with the capillary channel (121), and a diameter of the exhaust hole (131) is smaller than a diameter of the through hole (123).

8. The liquid sampling device (100) according to claim 7, wherein the gripping portion (110) is provided with an exhaust groove (111), and the exhaust groove (111) is in communication with the exhaust hole (131).

9. The liquid sampling device (100) according to claim 1, wherein a width of the slit (122) is b, where 0.01 mm ≤ b ≤ 0.5 mm.

10. The liquid sampling device (100) according to claim 1, wherein the capillary channel (121) comprises a collection section (1211) and a flow limiting section (1212) that are in communication to each other, the collection section (1211) is in communication with the through hole (123), and a diameter of the flow limiting section (1212) is smaller than a diameter of the collection section (1211).
